# EUROPEAN PATENT SPECIFICATION

(11) **EP 0 294 804 B1**
(45) Date of publication and mention of the grant of the patent: **29.09.1993**
(21) Application number: 88109201.9
(22) Date of filing: 09.06.1988
(51) Int. Cl.: C12Q 1/34

(54) **Method for determination of NAGase and reagent therefor**
Verfahren zur Bestimmung der NAGase und Reagens dafür
Procédé pour la détermination de la NAGase et réactif à cet effet

(30) Priority: 11.06.1987 JP 146143/87
(43) Date of publication of application: 14.12.1988
(73) Proprietor: SHIONOGI SEIYAKU KABUSHIKI KAISHA, Osaka 541 (JP)
(72) Inventor: Noto, Akira, Takatsuki-shi Osaka (JP); Nakajima, Kunihiro, Kashihara-shi Nara (JP); Sasakura, Kazuyuki, Yamatokooriyama-shi Nara (JP); Sugasawa, Tsutomu, Kobe-shi Hyogo (JP)
(74) Representative: VOSSIUS & PARTNER

(56) References cited:
- EP-A- 0 060 793
- EP-A- 0 097 506
- EP-A- 0 146 866
- EP-A- 0 180 961
- CLINICAL CHEMISTRY, vol. 34, no. 10, 1988; pp. 2140-2143#
- RÖMPPs CHEMIE-LEXIKON, 8th ed., 1979, Franck'sche Verlagshandlung, Stuttgart (DE); p. 493#

## Description

The present invention provides kits for the determination of N-acetyl-β-D-glucosaminidase (hereinafter referred to as NAGase) activity, which is one of the important markers of renal dysfunction, and methods for determining the activity by using them.

4-Methylumbelliferyl N-acetyl-β-D-glucosaminide (hereinafter referred to as 4MU-NAG) and p-nitrophenyl N-acetyl-3-D-glucosaminide (hereinafter referred to as PNP-NAG) have long been known as substrates for determining NAGase activity. These methods have, however, disadvantages such as requiring urine blanks and substrate blanks in each test. They are liable to be affected by inhibitors in urine, and they need a long time for the determination.

Sodio-meta-cresolsulfonphthaleinyl N-acetyl-β-D-glucosaminide (hereinafter referred to as MCP-NAG), wherein metacresolsulfonphthaleine (hereinafter referred to as MCP) is a color indicator, was developed and disclosed in KOKAI 58-994 as a substrate with less disadvantages than the above. It still has, however, disadvantages in that it requires a substrate blank and a terminating step to determine NAGase activities with addition of an alkaline agent (one-point method).

Recently, 2-chloro-4-nitrophenyl N-acetyl-β-D-glucosaminide (hereinafter referred to as CNP-NAG) was developed and disclosed in KOKAI 61-112092 as a reagent for determining NAGase activity without termination of the reaction (continuous method). In this method, enzyme activity is monitored continuously and the method can be easily adapted to automatic analysers. This still has a disadvantage, however, in that it takes several minutes to dissolve CNP-NAG completely, even in the presence of surfactants, because the substrate is hardly soluble in water. Further, it takes a long time to determine the activity of trace amounts of NAGase. Additionally, EP-0 146 866 describes sulfonphthaleinyl-β-D-galactosides as substrates for the enzyme β-D-galactosidase which can be colourmetrically determined upon substrate cleavage.

An object of the invention is to provide a new substrate for the determination of NAGase activity which is highly soluble and easy to handle. Another object of the invention is to provide kits for the determination of NAGase activity which require no urine blanks and can determine the activity by the continuous method. The determination of NAGase activity should be Precise and quick even if only very small amounts of NAGase are present. Thus, another object of the invention is to provide a very sensitive and quick method.

The present invention provides kits for determining NAGase activity comprising the following reagents (a) and (b):
(a) a substrate reagent containing sodio-3,3'-dichlorophenolsulfonphthaleinyl N-acetyl-β-D-glucosaminide (hereinafter referred to as CPR-NAG) of the formula: 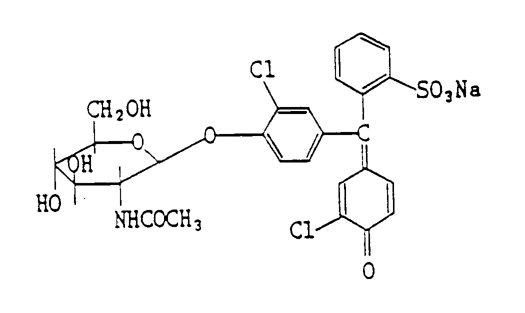 and
(b) a buffer reagent keeping the reaction pH between about 4.5 and about 8.0 wherein said kit does not contain an alkaline reagent for terminating the enzyme reaction.

### BRIEF DESCRIPTION OF THE DRAWINGS

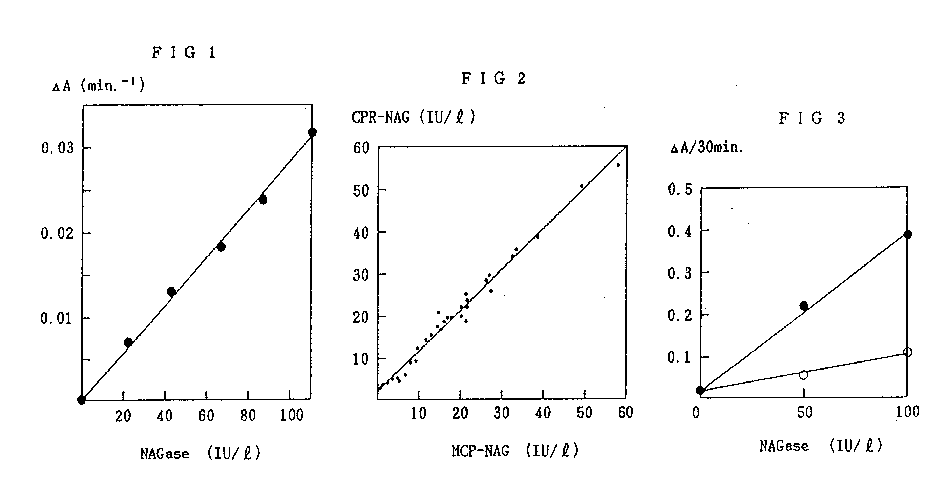

Figure 1 is a calibration curve obtained from NAGase with known activity and shows the relationship between the NAGase activity and 3,3'-dichlorophenolsulfonphthalein (chlorophenol red: hereinafter referred to as CPR) yielded. The abscissa indicates concentrations of NAGase (IU/ℓ) and the ordinate indicates the production rate of CPR determined by the rate of change in absorbance (△A₅₄₀nm/min).

Figure 2 shows the correlation between the MCP-NAG method and the CPR-NAG method of the present invention. The abscissa and ordinate indicate NAGase concentrations (IU/ℓ) obtained by the MCP-NAG method and the CPR-NAG method, respectively.
- Linear Regression:: Y = 0.97x + 1.84
- Coefficient of Correlation:: r = 0.99
Figure 3 shows calibration curves for the CPR-NAG method (the present invention) and the CNP-NAG method (prior art). The abscissa indicates concentration of NAGase (IU/ℓ) and the ordinate indicates increase of absorbance after 30 minutes, wherein the curve ●-● is for the CPR-NAG method (△A₅₄₀nm/30min.) and ○-○ for the CNP-NAG method (△A₄₀₅nm/30min.).

### Means to Resolve the Problem

Sodio-3,3′-dichlorophenolsulfonphthaleinyl N-acetyl-β-D-glucosaminide (CPR-NAG) used in the present invention can readily be prepared from 3,3′-dichlorophenolsulfonphthalein (CPR) and 1-chloro-1-deoxy-2,3,4,6-tetraacetyl-α-D-glucosamine, according to the process disclosed in KOKAI 58-994.

Unlike CNP-NAG, the aforementioned CPR-NAG is freely and quickly soluble in water and, therefore, no specific additives such as surfactants are needed. Accordingly, substrate solutions can be prepared very quickly.

It is very important to stabilize substrates, because their decomposition increases experimental error. CPR-NAG used in the present invention is fairly stable even in the absence of any stabilizers, and therefore, it may be formulated as it is. Further, it can be stored in a refrigerator for a long period of time, without significantly decomposing. Borax may be added to it as a stabilizer, if much longer shelf-life is desired. Borax may be added to one part of CPR-NAG at an amount of about 0.02 to about 2.0 parts, more preferably to about 0.6 to 1.2 parts.

In formulating the reagents into a kit, the substrate reagent and the buffer reagent may be prepared as either separate formulations or a single formulation. It is recommended to prepare the formulation as lyophilizate in view of its appearance and stability.

Although NAGase shows maximum activity at a pH value of about 4.5 to about 5.0 in the present invention using CPR-NAG, the apparent reaction rate reaches a maximum at a pH value of about 6.25. Accordingly, any buffer reagent may be used as long as it can keep the reaction pH between about 4.5 and about 8.0. Preferably, buffers which can keep the pH between about 6.0 and 6.5, and most preferably, those which can keep the pH between about 6.2 and 6.3 may be used. For example, a citrate buffer, a borax-citrate buffer, a citrate-phosphate buffer, a phosphate buffer, a borax-phosphate buffer, a sodium barbitate-sodium acetate buffer, Good's buffer and the like may be used.

In the method using the aforementioned MCP-NAG, the enzyme should be well reacted with the substrate and an alkaline reagent is added for enzyme inactivation and color development. This method thus requires an additional step compared to the present method. Further, since the color development cannot be observed along the course of the reaction, the determination may have to be repeated if the alkaline reagent is added at a time before the reaction proceeds well.

By using the kit of the present invention such a mistake can not occur as the course of the enzyme reaction can be traced directly by the change of the absorbance. As most analytical instruments are designed for continuous methods, it is quite easy to determine the enzyme activity automatically.

### (Principle of Measurement)

The substrate reagent CPR-NAG is hydrolyzed by NAGase to give CPR in a buffer solution at a pH of about 4.5 to 8.0. The resulting CPR, whose pKa is about 5.8, develops a reddish purple color immediately. NAGase activity is determined by the rate of change of absorbance with time.

### (Procedure for Continuous Method)

To 1.0 ml of a substrate buffer (2-6 mM CPR-NAG, pH about 4.5-8.0) 50 µl of a test sample* is added. The absorbances A¹ and A² of the mixed solution at a wavelength of 575 nm are measured by a spectrophotometer equipped with a constant-temperature cell at a time t¹ and a time t², respectively, after addition of the test sample.

Note:
The test sample* means urine or serum collected from human or animals, which is preferably measured immediately after the collection. If the sample has to be stored for a long time, it is recommended that the sample pH is adjusted to 6.5 with 2N-HCl or 2N-KOH. Under such a condition, NAGase activity in the test sample is stable for one day at room temperature and for 4 months at -20°C.

### (Definition of the Enzyme Activity)

Under a measuring condition, the amount of NAGase capable of producing 1 µmol of CPR per minute is defined to be 1 IU.

### (Calculation of the Enzyme Activity)

NAGase activity can be calculated according to the following equation. 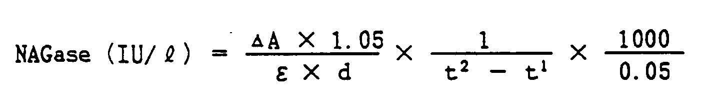
$\text{△A = A² - A¹}$

- ε =: the extinction coefficient (cm²/µM) of CPR at the pH and wavelength employed
- d =: the length of optical path (cm)
△t, i.e. (t²-t¹) is normally 1 to 20 minutes and preferably 5 to 10 minutes.

The present invention is explained in greater detail by the following Examples and Experiments. PREPARATION 1 Preparation of 3,3'-dichlorophenolsulfonphthaleinyl N-acetyl-β-D-glucosaminide pentaacetate (1)

To a stirred solution of chlorophenyl red (1.4g, 3.3mmol) in methanol (4ml), sodium methoxide (6.8ml, 3.3x2mmol) is added and the solution is stirred for further 15 minutes. The solvent is removed under reduced pressure below 35°C and the resulting residue is triturated with toluene and the solvent is removed under reduced pressure. After repeated co-distillation with toluene (^{x}2), the residue is dried under reduced pressure.

To a solution of the disodium salt obtained above in 7ml of dimethylformamide (DMF), acetocoloroglucosamine (1.1g, 3mmol) is added and the solution is stirred for 21 hours. Then the solution is combined with 10ml of Amberlite® IRC-50 (Röhm & Haas Co.) and the resulting mixture is stirred for 30 minutes. The resin is filtered off and washed with methanol. The filtrates and washings are collected and evaporated to dryness.

The residue is dissolved in pyridine (20ml) followed by addition of acetic anhydride (10ml) and the solution is allowed to stand at room temperature overnight. After removal of the solvent and reagent *in vacuo*, toluene is added to the residue and the solution is evaporated again to dryness *in vacuo*. The residue is chromatographed on silica gel (10g of SiO₂ containing 3% water, 0.063-0.2mmφ ) to give 2.1g of a colorless gummy matter from the dichloromethane fractions (160ml). This is purified again on column chromatography (Lober B, solvent system: acetic acid) to give 0.98g of the objective compound in the fourth to ninth fractions (15g per fraction). This is recrystallized from ethyl acetate/ether to give 0.8g (36.9% yield) of the captioned compound (1), mp. 142-143°C.
IR ν max(Nujol): 3265, 3090, 1750, 1663, 1604, 1556, 1352 cm⁻¹.
NMR (90MHz, CDCl₃) δ: 1.85(3H, s), 2.00(9H, s), 2.32(3H, s).

| Elementary Analysis Calcd. for C₃₅H₃₃NO₁₄Cl₂S·½H₂O: | | | | | | | | | | |
|---|---|---|---|---|---|---|---|---|---|---|
| | C; | 52.32, | H; | 4.27, | N; | 1.74, | Cl; | 8.83, | S; | 3.99. |
| Found: | C; | 52.33, | H; | 4.25, | N; | 1.73, | Cl; | 8.34, | S; | 3.84. |

### PREPARATION 2

### Preparation of sodio-3-3′-dichlorophenolsulfonphthaleinyl N-acetyl-β-D-glucosaminide (2)

To a stirred solution of 2.057g (2.56mmol) of the compound (1) prepared in Preparation 1 in 18ml of methanol, a solution of 0.9717M sodium methoxide (2.58x2mmol) in methanol (5.3ml) is added. After standing for 3 hours under nitrogen atmosphere, Amberlite® IRC-50 (10ml) is added and the mixture is stirred for an hour, then the resin is filtered off and washed with methanol. The filtrates and washings are collected and evaporated to dryness *in vacuo* at a temperature below 35°C to give a yellow foam. This is dissolved in 5 ml of water, chromatographed on DEAE-Sepharose® CL-6B (10ml; Pharmacia Co.), and the fractions are collected. The fractions are lyophilized to give 1.735g (98.1% yield) of the objective command (2) as an amorphous powder.

| Elementary Analysis Calcd. for C₃₅H₃₃NO₁₄Cl₂S·½H₂O: | | | | | | | | | | |
|---|---|---|---|---|---|---|---|---|---|---|
| | C; | 49.24, | H; | 4.28, | N; | 2.03, | Cl; | 10.26, | S; | 4.64. |
| Found: | C; | 48.87, | H; | 4.61, | N; | 2.05, | Cl; | 9.81, | S; | 4.38. |

### EXAMPLE 1

CPR-NAG is dissolved in water containing 50mM borax to prepare an aqueous solution containing 30mM CPR-NAG, 2ml portions of which are placed in 20ml glass vials and lyophilized to give a substrate reagent.

Dipotassium citrate (502.9g) and 1014.0g of potassium citrate are ground into a powder, mixed together, uniformed in particle size. 304.4mg portions of the mixture are then placed into a 25 ml plastic bottle to give a buffer reagent.

Distilled water (20ml) is added to the buffer reagent to prepare a buffer solution, which is then added to the substrate reagent to give a reagent solution (pH 6.25).

### EXAMPLE 2

In 500mM (pH 6.10) of a Bis-Tris* buffer solution 30mM CPR-NAG, 50mM borax, and 1.5M sodium chloride are dissolved and 2ml portions of the solution are placed in 20ml glass vials and then lyophilized to give a reagent for measurement containing a buffer reagent.
NOTE) Bis-Tris* means bis(2-hydroxyethyl)iminotris(hydroxymethyl)methane.

Distilled water (20ml) is added thereto to give a reagent solution for measurement (pH 6.25).

### EXAMPLE 3

30mM CPR-NAG is dissolved in 500mM potassium citrate buffer (pH 5.84) containing 50mM borax, and the solution is divided into 1.1ml portions and placed into 14ml glass vials and lyophilized.

11 ml of distilled water are added to the lyophilized substrate to give a substrate solution (pH 6.25).

### EXPERIMENT 1

In each well of a 96-well flat-bottomed micro titerplate 100 µl of the reagent solution (pH 6.25) prepared in Example 1 is placed and 10 µl each of a test sample is added thereto. The absorbance A¹ at the wavelength of 540nm is measured by a microplate spectrophotometer (type MCC 340, Titertech Co.). Samples to be tested are allowed to stand at room temperature and, precisely ten minutes later, A² at 540nm is measured again.

Absorbances $\text{(△B = B²-B¹)}$ of the blank, which is prepared by adding 10 µl of distilled water in place of the test sample, are measured in the same manner as above.

In the same manner as for the test sample, a calibration curve is obtained from the absorbances of the NAGase specimen with known activity. NAGase activity is calculated from the changes of absorbance per minute. 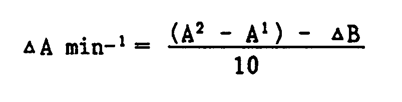
Figure 1 shows the calibration curve and Figure 2 shows correlation between the method of the present invention and the one-point method by the conventional MCP-NAG test (NAG Test Shionogi: trade name).

### EXPERIMENT 2

Using a commercialy available kit for NAGase activity (CNP-NAG continuous method), the reagent solution is prepared according to the recommended usage. The reagent solution (50 µl each) is placed in wells of a 96-well flat-bottomed micro titerplate. To the wells 10 µl each of standard solution of NAGase with known activity is also added. The absorbance A¹ at a wavelength of 405nm is measured by a microplate spectrophotometer (type MCC 340, Titertech Co.). Samples tested are allowed to stand at room temperature and, precisely 30 minutes later, A² at 405nm is measured again to give a calibration curve (○-○) for the CNP-NAG method (Figure 3).

The reagent solution (100 µl each, pH 6.25) prepared in Example 1 is placed into the wells of a 96-well flat-bottom micro titerplate and 10 µl each of the test sample is also added thereto. The absorbance A¹ at 540nm is measured by a microplate spectrophotometer (type MCC 340, Titertech Co.). Samples tested are allowed to stand at room temperature and, precisely 10 minutes later, A² at 540nm is measured to give calibration curve (●-●) for the CPR-NAG method of the present invention (Figure 3).

As clearly indicated in Figure 3, the method of the present invention is approximately 3 to 4 times more sensitive than the conventional method. If the concentration of NAGase is very low, methods with low sensitivities require a long period of time to give a precise determination; while those with high sensitivities which the present invention provides can give a precise detemination in a very short time. This is advantageous.

## Claims

1. A kit for determining N-acetyl-β-D-glucosaminidase activity comprising the following reagents (a) and (b):
(a) a substrate reagent containing sodio-3,3'-dithlorophenolsulfonphthaleinyl N-acetyl-β-D-glucosaminide of the formula: 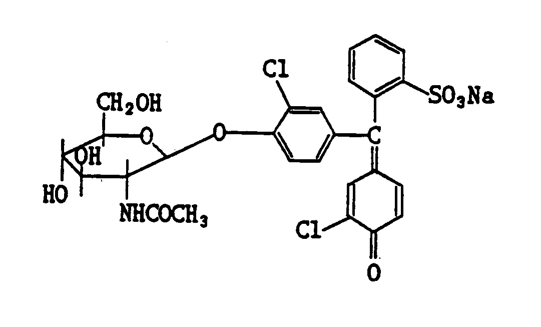 and
(b) a buffer reagent keeping the reaction pH between about 4.5 and about 8.0, wherein said kit does not contain an alkaline reagent for terminating the enzyme reaction.

2. The kit claimed in Claim 1, wherein said reagents (a) and (b) are placed together in a single container.

3. The kit claimed in Claim 2, comprising a lyophilized mixture of sodio-3,3'-dichlorophenolsulfonphthaleinyl N-acetyl-β-D glucosaminide, borax as a stabilizer, and a buffer reagent.

4. The kit claimed in Claim 2 or 3, wherein the buffer reagent is a citrate buffer.

5. The kit claimed in Claim 1, wherein said reagents (a) and (b) each is placed in individual containers.

6. The kit claimed in Claim 5, wherein said substrate reagent (a) is a lyophilized mixture of sodio-3,3'-dichlorophenolsulfonphthaleinyl N-acetyl-β-D-glucosaminide and borax as a stabilizer.

7. A method for determining N-acetyl-β-D-glucosaminidase activity which comprises dissolving a reagent containing sodio-3,3'-dichlorophenolsulfonphthaleinyl N-acetyl-β-D-glucosaminide of the following formula: 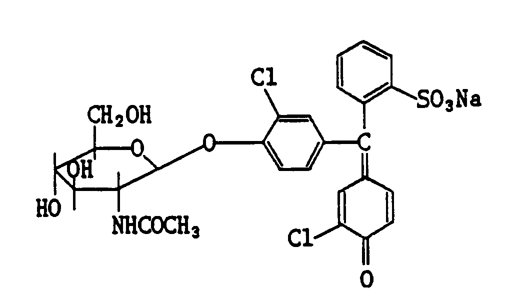 in a buffer to give a substrate solution of pH 6.0 to pH 6.5, adding a test sample to the substrate solution, and colorimetrically measuring the resulting 3,3,-dichlorophenolsulfonphthalein continuously wherein said method does not comprise the addition of an alkaline reagent for terminating the enzyme reaction.

## Patentansprüche

1. Kit zur Bestimmung von N-Acetyl-ß-D-glucosaminidase-Aktivität, umfassend die folgenden Reagenzien (a) und (b):
(a) ein Substratreagenz, enthaltend Natrium-3,3'-dichlorphenolsulfonphthaleinyl-N-acetyl-β-D-glucosaminid der Formel: 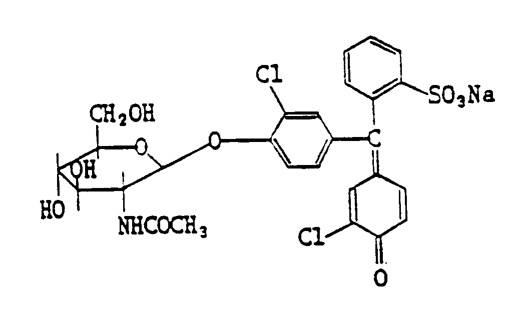 und
(b) ein Pufferreagenz, das den pH-Wert der Reaktion zwischen etwa 4,5 und etwa 8 hält,
wobei der Kit kein alkalisches Reagenz zur Beendigung der Enzymreaktion enthält.

2. Kit nach Anspruch 1, wobei die Reagenzien (a) und (b) sich zusammen in einem einzigen Behälter befinden.

3. Kit nach Anspruch 2, umfassend ein gefriergetrocknetes Gemisch von Natrium-3,3'-dichlorphenolsulfonphthaleinyl-N-acetyl-β-D-glucosaminid, Borax als Stabilisator und ein Pufferreagenz.

4. Kit nach Anspruch 2 oder 3, wobei das Pufferreagenz ein Citratpuffer ist.

5. Kit nach Anspruch 1, wobei die Reagenzien (a) und (b) sich jeweils in individuellen Behältern befinden.

6. Kit nach Anspruch 5, wobei das Substratreagenz (a) ein gefriergetrocknetes Gemisch aus Natrium-3,3'-dichlorphenolsulfonphthaleinyl-N-acetyl-β-D-glucosaminid und Borax als Stabilisator ist.

7. Verfahren zur Bestimmung von N-Acetyl-β-D-glucosaminidase-Aktivität, umfassend das Lösen eines Reagenzes, das Natrium-3,3'-dichlorphenolsulfonphthaleinyl-N-acetyl-β-D-glucosaminid der folgenden Formel enthält: 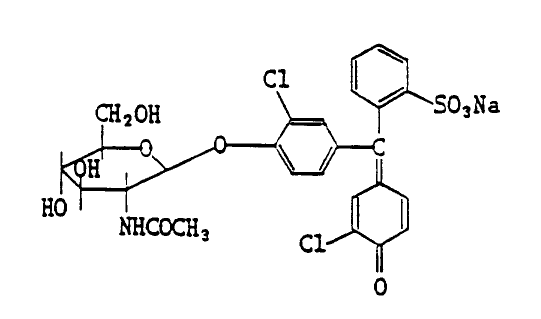 in einem Puffer, wobei eine Substratlösung mit einem pH-Wert von 6,0 bis 6,5 erhalten wird, Zugeben der Testlösung zur Substratlösung und kontinuierliche colorimetrische Messung des erhaltenen 3,3'-Dichlorphenolsulfonphthaleins, wobei die Methode nicht die Zugabe eines alkalischen Reagenzes zur Beendigung der Enzymreaktion umfaßt.

## Revendications

1. Trousse pour déterminer l'activité de la N-acétyl-β-D-glucosaminidase, comprenant les réactifs (a) et (b) suivants:
(a)un réactif formant substrat contenant du sodio-3,3'-dichlorophénolsulfonphtaléinyl N-acétyl-β-D-glucosaminide de la formule : 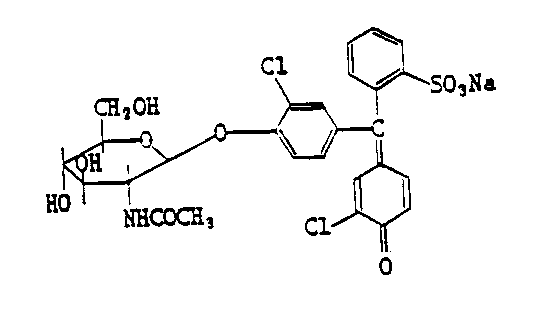 et
(b) un réactif formant tampon maintenant le pH de la réaction entre environ 4,5 et environ 8,0, ladite trousse ne contenant pas de réactif alcalin pour terminer la réaction enzymatique.

2. Trousse suivant la revendication 1, caractérisée en ce que les réactifs (a) et (b) sont places ensemble dans un seul récipient.

3. Trousse suivant la revendication 2, caractérisée en ce qu'elle comprend un mélange lyophilisé de sodio-3,3'-dichlorophénolsulfonphtaléinyl N-acétyl-β-D-glucosaminide, de borax comme stabilisant et d'un réactif formant tampon.

4. Trousse suivant l'une ou l'autre des revendications 2 et 3, caractérisée en ce que le réactif formant tampon est un tampon de citrate.

5. Trousse suivant la revendication 1, caractérisée en ce que les réactifs (a) et (b) sont placés chacun dans des récipients individuels.

6. Trousse suivant la revendication 5, caractérisée en ce que le réactif formant substrat (a) est un mélange lyophilisé de sodio-3,3'-dichlorophénolsulfonphtaléinyl N-acétyl-β-D-glucosaminide et de borax comme stabilisant.

7. Procédé pour déterminer l'activité de la N-acétyl-β-D-glucosaminidase, qui comprend la dissolution d'un réactif contenant du sodio-3,3'-dichlorophénolsulfonphtaléinyl N-acétyl-β-D-glucosaminide de la formule suivante : 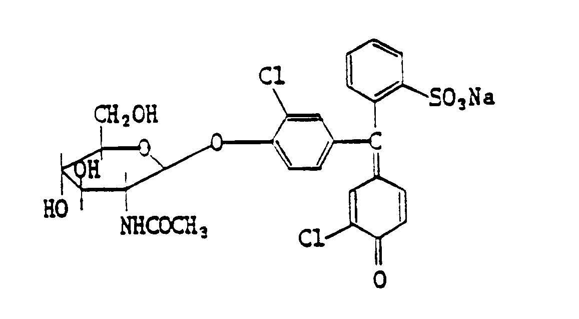 dans un tampon pour obtenir une solution de substrat de pH 6,0 à pH 6,5, l'addition d'un échantillon d'essai à la solution de substrat et la mesure colorimétrique continue de la 3,3'-dichlorophénolsulfonphtaléine résultante, ledit procédé ne comprenant pas l'addition d'un réactif alcalin pour terminer la réaction enzymatique.
